# FASCICULE DE BREVET EUROPEEN

(11) **EP 0 649 274 B1**
(45) Date de publication et mention de la délivrance du brevet: **03.02.1999**
(21) Numéro de dépôt: 94915208.6
(22) Date de dépôt: 04.05.1994
(51) Int. Cl.: A01N 37/16

(54) **COMPOSITIONS AQUEUSES COMPRENANT UN PEROXYACIDE ORGANIQUE**
ORGANISCHE PEROXYSÄURE ENTHALTENDE WÄSSRIGE ZUSAMMENSETZUNGEN
AQUEOUS COMPOSITIONS COMPRISING AN ORGANIC PEROXIACID

(30) Priorité: 05.05.1993 FR 9305376
(43) Date de publication de la demande: 26.04.1995
(73) Titulaire: CHEMOXAL SA, 75321 Paris 07 (FR)
(72) Inventeur: NICOLLE, Rémy, F-92190 Meudon (FR); LE ROUZIC, Daniel, F-95120 Ermont (FR); CRISINEL, Pascal, F-78000 Versailles (FR); DECLERCK, Gérard, F-95210 Saint-Gratien (FR); LEDON, Henry, F-78000 Versailles (FR)
(74) Mandataire: Conan, Philippe Claude
(86) Numéro de dépôt international: FR9400517
(87) Numéro de publication internationale: WO9424863

(56) Documents cités:
- EP-A- 0 252 278
- EP-A- 0 421 974
- GB-A- 2 255 507
- PHARMAZIE, vol.31, 1976, BERLIN DD pages 117 - 120 H. KUHN ET AL. 'versuche zur antimikrobiellen behandlung von dermatologischen zubereitungen mittels peressigsäure'
- CHEMICAL ABSTRACTS, vol. 79, no. 25, 24 Décembre 1973, Columbus, Ohio, US; abstract no. 143186g, H.P. WERNER ET AL 'destruction of spores in alcohol by peracetic acid' & ZENTRALBL. BAKTERIOL. PARASITENK., vol.157, no.4, 1973 page 387-91
- CHEMICAL ABSTRACTS, vol. 104, no. 1, 6 Janvier 1986, Columbus, Ohio, US; abstract no. 2072q, E. FUSTES ET AL. 'teat profylactic disinfection against intramammary infections.' & REV. SALUD ANIM., vol.7, no.2, 1985 page 193-200
- CHEMICAL ABSTRACTS, vol. 104, no. 25, 23 Juin 1986, Columbus, Ohio, US; abstract no. 221896q, E. FUSTES ET AL. 'teat disinfection for the prevention of intramammary infections' & REV. SALUD ANIM., vol.7, no.3, 1985 pages 323 - 332

## Description

La présente invention concerne des compositions aqueuses désinfectantes, stables dans le temps, non irritantes et présentant un large spectre antimicrobien, ainsi que leurs utilisations, notamment en tant qu'agent d'hygiène.

De nombreuses compositions destinées à la désinfection de la peau ou des muqueuses ont déjà été commercialisées. De telles compositions peuvent se présenter sous forme liquide et comportent à titre d'agent désinfectant ou antiseptique, des composés ammonium quaternaire tels les dérivés du chlorhexidine comme le digluconate de chlorhexidine, ou des composés tels le parachlorométhacrésol ou le trichlorocarbanilide

Habituellement ces compositions comportent divers agents tensio-actifs en vue notamment de favoriser le nettoyage de la peau. Elles sont alors communément dénommées "savon liquide antiseptique". Ces compositions ont cependant pour principaux inconvénients, d'une part, de présenter un spectre antimicrobien limité à certaines populations microbiennes choisies généralement parmi des bactéries Gram+ ou Gram-, des micromycètes, tels des levures ou des moisissures, ou des virus, et, d'autre part, de ne permettre qu'une faible diminution en nombre d'une population microbienne donnée, c'est-à-dire de ne présenter qu'un pouvoir désinfectant ou antiseptique réduit. Pour pallier ce dernier inconvénient, il est habituellement recommandé de laisser la composition désinfectante en contact avec la peau ou la muqueuse à désinfecter pendant plusieurs minutes ; mais même ainsi, la diminution de la population microbienne reste souvent insuffisante. De plus, dans la pratique courante, il a pu être constaté qu'un utilisateur ne pratique que rarement un temps de contact supérieur à une minute, ce temps de contact étant plus généralement de l'ordre de 30 secondes.

Un besoin s'est donc fait sentir pour des compositions désinfectantes présentant un large spectre antimicrobien, un fort pouvoir désinfectant se manifestant rapidement, en tout cas en moins de une minute et de préférence en moins de 30 secondes.

Par ailleurs, il est connu que les peroxyacides organiques présentent un pouvoir désinfectant ou antimicrobien. Les peroxydes organiques sont habituellement utilisés pour la désinfection des sols et autres surfaces. On les utilise également pour la désinfection d'articles destinés à entrer en contact avec un milieu biologique. Ainsi, des solutions aqueuses de peroxyacides ont été décrites dans la demande de brevet EP-A-472.713 comme agent désinfectant de cuves de lait, dans la demande de brevet EP-A-370.850 comme agent désinfectant de dispositifs pour hémodialyse ou dans les demandes de brevet GB 2.255.507 et EP-A-421.974 comme désinfectant des gros volumes difficilement immersibles et des surfaces non horizontales. En vue de la désinfection de tels articles, la concentration en peroxyacide de l'agent désinfectant est toujours supérieure à 0,1 % en poids, et plus généralement supérieure à 1 % en poids. En raison notamment de leur caractère agressif et irritant, il convient d'éliminer soigneusement par lavage à l'eau toute trace de peroxyacides des articles désinfectés avant leur mise en contact avec un milieu biologique comme le lait ou le sang. C'est aussi en raison de leur caractère agressif et irritant pour la peau et les muqueuses que, jusqu'à présent, à la connaissance de la demanderesse aucune composition désinfectante destinées notamment à l'hygiène corporelle de l'homme ou de l'animal n'a été commercialisée.

Par ailleurs, la demanderesse a pu constater qu'en raison de leur fort pouvoir oxydant, les peroxyacides organiques peuvent dégrader d'autres additifs, tels des agents tensio-actifs, qu'il convient souvent d'incorporer dans une composition du type savon liquide antiseptique. A l'inverse, certains tensio-actifs peuvent parfois rapidement dégrader des peroxyacides organiques et leur faire perdre leurs propriétés désinfectantes.

La demanderesse a également pu constater que ces compositions comportant certains additifs tels des agents tensio-actifs et des peroxyacides organiques, si elles présentent un aspect homogène pendant une courte période, peuvent se dissocier en au moins deux phases, celà au bout de quelques mois, voire quelques semaines.

Or des compositions destinées à un usage humain ou animal, pouvant être amenées à être stockées pendant plusieurs mois, voire plus d'un an avant d'être utilisées, doivent conserver un aspect homogène pendant une telle période.

Un premier objet de l'invention consiste alors en des compositions désinfectantes présentant un large spectre antimicrobien ainsi qu'un fort pouvoir antimicrobien ; celui-ci pouvant se manifester après un temps de contact court. Les compositions de l'invention sont par ailleurs non agressives et non irritantes pour la peau et les muqueuses de l'homme et de l'animal, et stables dans le temps, c'est-à-dire qu'elles restent homogènes et peuvent conserver un pouvoir désinfectant même après un stockage prolongé, ceci en présence ou non d'additifs tels des tensio-actifs.

Par ailleurs, ces compositions présentent une viscosité telle qu'elles peuvent être utilisées comme savon liquide désinfectant ou antiseptique, ou comme agent d'hygiène des trayons.

Un second objet de l'invention consiste en l'utilisation de ces compositions comme agent d'hygiène pour la désinfection de la peau ou des muqueuses de l'homme ou de l'animal.

La présente invention consiste en une composition aqueuse stable dans le temps, caractérisée en ce qu'elle comporte :
- un peroxyacide organique en une concentration inférieure à 0,09 % en poids ;
- et au moins un agent épaississant en une concentration telle que la viscosité de la composition est supérieure à 100 mPa.s (Brookfield LVT 2 à 20°C).

Les peroxyacides organiques peuvent être plus particulièrement choisis dans le groupe constitué par :
1) un monoperoxyacide de formule (I) :

   R₁ - CO₃H (I)

   dans laquelle R₁ représente un radical alkyle en C₁-C₂₄ linéaire ou ramifié, un radical aryle ou un radical cycloalkyle en C₃-C₁₀, et
2) un diperoxyacide de formule (II) :

   H₃OC - R₂ - CO₃H (II)

   dans laquelle R₂ représente un radical alkylène en C₁-C₂₄ linéaire ou ramifié, un radical arylène ou un radical cycloalkylène en C₃- C₁₀,
   chacun de R₁ ou de R₂ étant substitué ou non par un ou plusieurs groupes fonctionnels ou radicaux.

Les radicaux aryles et arylènes selon l'invention peuvent comporter de 1 à 5 noyaux benzéniques, de préférence un noyau benzénique, qui peuvent le cas échéant être fusionnés à un ou plusieurs, généralement 1 ou 2 cycles comprenant 5 ou 7 atomes de carbone.

Une composition selon l'invention est considérée comme stable dans le temps quand sa concentration en peroxyacide varie de moins de 10 % (en poids) sur une période de temps supérieure à 6 mois, de préférence supérieure à 12 mois, à compter de la fin du temps de mûrissement de ladite composition. Ce temps de mûrissement est explicité ci-après.

En vue de maintenir la stabilité d'une composition selon l'invention sur une telle période de temps, il peut être tout à fait souhaitable d'éviter la présence de monoalcools, tels que l'éthanol ou l'isopropanol. La demanderesse a en effet pu constater que la présence d'un monoalcool induisait une diminution notable de la stabilité, et donc de la teneur en peroxyacide de la composition selon l'invention.

Dans le cadre de la présente invention et sauf indication contraire, lorsque des radicaux ou des restes sont substitués, les substituants sont de préférence choisis dans le groupe constitué par les radicaux ou groupes fonctionnels suivants :
- un groupe carboxylique libre sous forme ester ou amide, ou salifié par un contre-ion alcalin, alcalino-terreux, ammonium ou phosphonium,
- un radical alkyle en C₁-C₂₄, linéaire ou ramifié, substitué ou non par une ou plusieurs , généralement une à cinq fonctions carboxyliques et/ou amines,
- un radical acyle de formule

   R - CO -

   R représentant un radical alkyle en C₁ - C₂₄, linéaire ou ramifié,
- un radical alkoxy en C₁-C₂₄ substitué ou non par une ou plusieurs, généralement une à cinq, fonctions carboxylique et/ou amine,
- un radical aryle,
- une fonction amine, hydroxyle, nitrile, nitro, trifluorométhyle, sulfonyle,
- un atome d'halogène tel le fluor, le chlore ou le brome.

A titre de monoperoxyacides de formule (I) préférés, on peut citer ceux où R₁ représente un radical alkyle en C₁-C₁₂. Parmi les peroxyacides organiques de formule (I) ou (II), on peut notamment citer les acides :
- peracétique,
- perpropionique,
- monoperoxyazélaïque,
- percaprylique,
- perundécylénique,
- perlaurique,
- monoperoxysuccinique,
- monoperoxyglutarique,
- diperoxysuccinique,
- diperoxyazélaïque,
- octyl-2-peroxybutanedioïque-1-4,
- décyl-2-peroxybutanedioïque-1,4,
- dodécyl-2-peroxybutanedioïque-1-4,
- diperoxy-dodécanedioïque-1,12 ;
   l'acide peracétique étant un peroxyde organique particulièrement préféré dans le cadre de la présente invention.

L'acide peroxybenzoïque et ses dérivés tels les acides métachloroperoxy-benzoïque, p-tertiobutylperoxy-benzoïque, p-nitroperoxybenzoïque, monoperphtalique ou aminophtaloylperacétique, peuvent également être avantageusement utilisés.

A titre de monoperoxyacide de formule (I), on peut également citer les imidoperoxyacides de formule (III) : 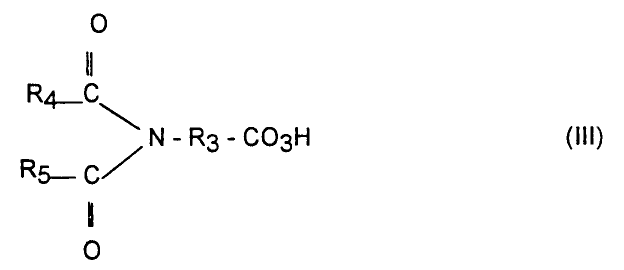 dans laquelle R₃ est un radical ayant l'une des significations de R2, R₃ étant substitué ou non par un ou plusieurs groupes fonctionnels ou radicaux et R₄ et R₅ sont identiques ou différents et représentent un radical alkyle en C₁-C₈ linéaire ou ramifié, substitué ou non par un ou plusieurs groupements fonctionnels ou radicaux, ou R₄ et R₅ ensemble forment un reste aliphatique en C₂-C₄, linéaire, saturé ou mono-insaturé, substitué ou non par un radical alkyle en C₁-C₂₄ linéaire ou ramifié.

Parmi les composés de formule (III), on préfère ceux comportant un hétérocycle azoté, de formule (IV) : 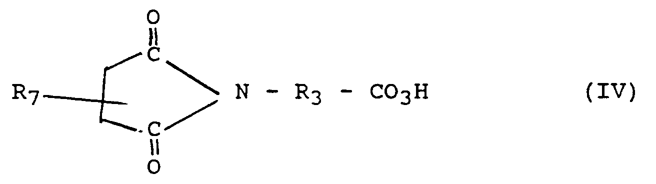 dans laquelle R₃ est un reste tel qu'indiqué plus haut, et R₇ est un radical alkyle en C₁-C₂₄, linéaire ou ramifié, substitué ou non par un ou plusieurs groupements fonctionnels ou radicaux, ou R₇ est un groupe aryle, fusionné avec l'hétérocycle azoté, substitué ou non par un ou plusieurs groupes fonctionnels ou radicaux.
Avantageusement, R₃ est un radical alkylène en C₁-C₂₀, de préférence en C₁-C₁₄ ; et R₇ est un radical phénylène fusionné avec l'hétérocycle azoté.

A titre d'exemple de tels composés peuvent être l'acide phtalimidoperacétique, l'acide phtalimidoperpropionique, l'acide pthalimidoperdodecanoïque ou l'acide phtalimidopercaproïque.

Les peroxyacides organiques pouvant être mis en oeuvre dans la composition selon l'invention peuvent être préparés de manière connue, par exemple selon les procédés décrits dans les demandes de brevet européen N° 24.219 et 441.235.

La concentration en peroxyacide organique dans la composition peut être comprise entre 0,001 % et 0,05 % en poids et de préférence entre 0,002 % et 0,025 % en poids.

Les agents épaississants mis en oeuvre dans le cadre de la présente invention sont les uréthannespolyéthers.

Ces composés sont décrits dans la demande de brevet FR-A-2.687.069, au nom de L'OREAL, en particulier page 2, ligne 19 à page 3, ligne 25, ainsi que dans la demande de brevet EP-A-260.430, au nom d'AKZO. Ces demandes de brevet sont intégrées ici pour référence.

Les uréthannes-polyéthers utilisables selon l'invention répondent à la formule (VI) suivante : 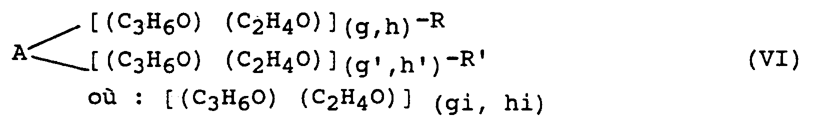 avec (gi, hi) désignant (g, h) ou (g', h'), signifie qu'il s'agit d'un polymère statistique d'oxyde de propylène et d'oxyde d'éthylène contenant h mole d'oxyde d'éthylène et g mole d'oxyde de propylène, réparties de manière aléatoire dans la chaîne polymérique ;
A désigne un reste de diisocyanate aliphatique, cycloaliphatique ou aromatique, de préférence un reste polyméthylène diisocyanate, toluène diisocyanate ou méthanediphényl diisocyanate ;
R et R', identiques ou différents, désignent un radical alkyle ou alcényle en C₈-C₃₀, de préférence en C₁₀-C₂₀ et plus particulièrement en C₁₂-C₁₈ ; gᵢ et hᵢ, identiques ou différents, étant tels que la somme de gᵢ+hᵢ varie de 20 à 200 moles et de préférence de 60 à 120 moles ; le rapport molaire hᵢ/gᵢ est compris entre 30/90 et 90/10 et plus particulièrement entre 70/30 et 85/15.

Les composés de formule (VI) plus particulièrement préférés sont ceux où A désigne le reste hexaméthylène diisocyanate, R et R' désignent un radical lauryle ou un mélange de radicaux dérivés du suif.

Les radicaux Rᵢ-[(C₂H₄O) - (C₃H₆O)]_{(gi, hi)} ont de préférence un poids moléculaire de l'ordre de 4000, avec Rᵢ désignant R ou R' et gᵢ et hᵢ désignent g ou h ou g' et h', tels que définis ci-dessus.

Les composés de formule (VI) peuvent être obtenus par réaction d'un diisocyanate avec un ou deux alcools gras polyoxyéthylénés et polyoxypropylénés, de formule Rᵢ[(OC₂H₄)-(OC₃H₆)]OH_{(gi hi)}, dans laquelle Rᵢ, gᵢ, hᵢ ont les significations indiquées ci-dessus, utilisé(s) en excès par rapport au diisocyanate afin que ce dernier soit totalement consommé.

Des agents viscosants de formule (VI) tout particulièrement préférés sont ceux commercialisés par la société AKZO sous le nom DAPRAL, tel le DAPRAL T210, le DAPRAL T212 ou le DAPRAL GT282.

Le poids moléculaire moyen des composes de formule (VI) peut être compris entre 1000 et 20000, de préférence entre 3000 et 10000.

Enfin, à titre d'autres agents viscosants utilisables, on peut citer les composés désignés dans C.T.F.A. International Cosmetic Ingredients Dictionary, sous le nom Ceteareth tel le Ceteareth-33 commercialisé par la Société Seppic sous le nom Cire de Lanol de CTO.

La concentration en agent viscosant dans la composition est de préférence telle que la viscosité de ladite composition est avantageusement comprise entre 100 et 700 mPa.s, plus préférentiellement comprise entre 150 et 700 mPa.s. Les valeurs de viscosité sont mesurées au moyen d'un Brookfield LVT2 à 20°C.

Les compositions selon l'invention peuvent en outre comporter un ou plusieurs tensio-actifs choisis parmi les tensio-actifs amphotères, cationiques, ou de préférence les tensio-actifs non-ioniques et anioniques.

Ces tensio-actifs peuvent conférer à la composition selon l'invention, un effet nettoyant moussant ou mouillant. Ils sont plus généralement utilisés dans des formulations du type savon liquide désinfectant ou dans des compositions destinées à l'hygiène des trayons.

De manière surprenante, la présence de ces tensioactifs n'entraîne pas une diminution de la stabilité dans le temps de la composition selon l'invention. Celle-ci reste donc active et présente un aspect homogène même lorsqu'elle est stockée plusieurs mois, voire plusieurs années.

A titre de tensio-actifs anioniques, on peut citer les alkyléthersulfates, les alkylbenzénesulfonates, les alkylsulfates, les oléfines sulfonates et les alkyléthercarboxylates, salifiés par un ou plusieurs contreions, tels un ion d'un métal alcalin, comme le sodium, ou le potassium, ou d'un métal alcalino-terreux. Un tensio-actif anionique préféré est le lauryléthersulfate de sodium.

A titre de tensio-actif non-ionique, on peut citer les esters d'acides gras de sorbitan, les esters d'acides gras de sorbitan polyoxyéthylé (POE) et/ou polyoxypropylé (POP) et, de préférence, les alkylpolyglycosides de formule (VII)

R₉O (R₁₀O)z (G)ₓ (VII)

où G est un reste d'un sucre réducteur contenant 5 ou 6 atomes de carbone, x est un nombre compris entre 1 et 10, R₉ est un radical aliphatique en C₆-C₂₄, linéaire ou ramifié, saturé ou insaturé, R₁₀ est un groupe alkylène ayant de 2 à 4 atomes de carbone, et z est compris entre 0 et 20.

Plus particulièrement, G peut être un reste glucose alors que x est compris entre 1 et 5, R₉ est un radical alkyle comprenant de 8 à 18 atomes de carbone et z est égal à zéro.

La concentration en tensio-actifs dans la composition peut être comprise entre 0,1 et 15 % en poids, de préférence entre 1 et 7 % en poids.

Les compositions selon l'invention peuvent en outre comporter d'autres additifs, tels des émollients, des colorants, des agents adoucissants et des agents séquestrants.

Par ailleurs, les peroxyacides organiques étant classiquement obtenus par réaction entre un acide organique et du peroxyde d'hydrogène, les compositions selon l'invention peuvent également comporter de tels composés. La concentration en peroxyde d'hydrogène dans la composition est habituellement inférieure à 8 % en poids, de préférence comprise entre 0,1 % et 3 %, en poids, la concentration en acide organique dans la composition est généralement inférieure à 1,5 % en poids, de préférence comprise entre 0,1 et 1 % en poids. Des agents stabilisants des peroxyacides organiques comme le pyrophosphate acide de sodium peuvent encore être contenus dans la composition.

Les compositions selon l'invention peuvent être préparées, par mélange, sous agitation, d'une solution aqueuse d'un peroxyacide organique prête à l'emploi avec l'agent épaississant et de l'eau, ainsi que le cas échéant, le ou les tensio-actifs et les autres additifs, mentionnés ci-dessus.

Alternativement, on peut préparer les compositions de l'invention en mélangeant sous agitation, du peroxyde d'hydrogène, un acide organique précurseur d'un peroxyacide organique choisi et les autres constituants de la composition.

Les teneurs en peroxyde d'hydrogène et en acide organique sont choisies de sorte qu'après un certain temps de mûrissement, on obtienne la concentration en peroxyacide organique désirée. Ce temps de mûrissement peut être compris entre 15 jours et six mois.

Ce temps de mûrissement peut être considérablement réduit lorsqu'on soumet les mélanges obtenus tel qu'indiqué ci-dessus, à une température comprise entre 30 et 45°C, de préférence entre 35 et 43°C. Ainsi le temps de mûrissement peut être inférieur à 3 jours.

Les compositions selon l'invention décrite ci-dessus peuvent être utilisées comme agent d'hygiène pour la désinfection de la peau ou des muqueuses de l'homme ou de l'animal.

Elles peuvent notamment être utilisées comme agent désinfectant et/ou nettoyant, notamment comme agent d'hygiène pour la désinfection des trayons. Pour cette utilisation, les compositions peuvent ne comporter que de faibles teneurs en peroxyacides, par exemple des teneurs comprises entre 0,001 % et 0,005 % en poids.

Les compositions selon l'invention peuvent encore être utilisées comme savon nettoyant et antiseptique ou antimicrobien, notamment sous forme liquide. Dans cette utilisation, les compositions renferment le plus souvent au moins un agent tensio-actif tel que défini plus haut.

Les compositions selon l'invention étant visqueuses présentent un grand intérêt dans ce type d'utilisation, dans la mesure où elles restent en contact intime avec la peau ou la muqueuse à désinfecter pendant le laps de temps requis, après quoi elles peuvent être éliminées par simple lavage à l'eau.

Il est important de noter que la viscosité de ces compositions restent stables pendant plusieurs mois, voire plusieurs années.

Les exemples qui suivent ont pour but d'illustrer la présente invention.

Dans ces exemples, les pourcentages sont en poids et les viscosités sont mesurées au moyen d'un Brookfield LVT2 à 40 tours/minutes à 20°C.

### Exemple 1 : Savon liquide désinfectant

Dans un réacteur de deux litres, agité mécaniquement, on introduit 500 g d'eau distillée puis :
- 0,2 g de pyrophosphate acide de sodium,
- 40 g de Dapral T 210 ¹
- 20 g de lauryléthersulfate de sodium
- 39 g de peroxyde d'hydrogène à 70 %
- 4,2 g d'acide acétique,
- 20 g de décylglucoside,
- 3,2 g d'une solution à 10 % en poids d'acide peracétique,
- q s p 1000 g d'eau distillée.

(1) : agent viscosant commercialisé par la société Akzo.

Après un temps de mise à l'équilibre de 1 mois à température ambiante, la teneur en acide peracétique de la composition atteint 0,01 %, la teneur en peroxyde d'hydrogène est de 2,8 % et la viscosité de la solution est de 430 mPa.s.

### Exemple 2 : Savon liquide désinfectant

Dans un réacteur de deux litres, agité mécaniquement, on introduit 500 g d'eau distillée, puis:
- 0,2 g de pyrophosphate acide de sodium,
- 40 g de Dapral T 210 ¹,
- 20 g de lauryléthersulfate de sodium,
- 39 g de peroxyde d'hydrogène à 70 %,
- 4,2 g d'acide acétique,
- 10 g de capryl - caprylylglucoside
   (rapport molaire capryle/caprylyle = 50/50)
- 3,2 g d'une solution à 10 % d'acide péracétique,
- q s p 1000 g d'eau distillée.

(1) : agent viscosant commercialisé par la Société Akzo.

Après un temps de mise à l'équilibre de 1 mois à température ambiante, la teneur en acide peracétique atteint 0,01%, la teneur en peroxyde d'hydrogène est de 2,8 % et la viscosité de la solution est de 280 mPa.s.

Les savons liquides désinfectants des exemples 1 et 2 ont été testés sur 3 lapins. Ils se sont avérés non irritants pour la peau et très faiblement irritants pour l'oeil.

L'appréciation de la tolérance oculaire a été effectuée selon la méthode officielle, définie par l'Arrêté du 3 Mai 1990, paru au Journal Officiel de la République Française du 14 Novembre 1990.

L'appréciation de la tolérance cutanée a été effectuée selon la méthode officielle, définie par l'arrêté du 1er Février 1982, paru au Journal Officiel de la République Française du 21 Février 1982.

### Exemple 3 : Savon liquide désinfectant :

Dans un réacteur de deux litres, agité mécaniquement, on introduit tout d'abord, 500 g d'une solution comprenant :
- 0,04 % d'acide peracétique,
- 1 % d'acide acétique,
- 5,6 % de peroxyde d'hydrogène,
- 0,04 % de pyrophosphate de sodium,
- q s p 500 g d'eau distillée,
   puis :
- 40 g de Dapral T 210 ¹
- 40 g de lauryléthersulfate de sodium,
- q s p 1000 g d'eau distillée
   (1):agent viscosant commercialisé par la Société Akzo.

Après un temps de mise à l'équilibre de 5 mois à température ambiante, la teneur en acide peracétique atteint 0,01%, la teneur en peroxyde d'hydrogène est de 2,8 %. Cette composition est stable pendant plus de 18 mois.

### Exemple 4 : Savon liquide désinfectant :

On procède comme dans l'exemple 3, mais en remplaçant les 40 g de lauryléthersulfate de sodium par 20 g de ce même tensio-actif et 10 g de caprylcaprylylglucoside (rapport molaire capryle/caprylyle = 50/50)

A l'équilibre, la teneur en acide peracétique est de 0,01% et celle en peroxyde d'hydrogène est de 2,8 %.

Cette composition est stable pendant plus de 18 mois.
Le pouvoir bactéricide des savons liquides des exemples 3 et 4 a été testé selon le protocole NFT 72-151 dans les conditions suivantes :
- température d'essais: 32 ° C
- bactéries testées :Staphylococcus aureus CNCM 53514
- temps de contact :1 min.
- dilution :pur et dilué au 1/2
- méthode par filtration sur membrane 0,45 µm
   Les deux savons liquides ont permis de diminuer par 10⁷ fois la population initiale de bactéries testées.

### Exemple 5 : Composition désinfectante pour l'hygiène des trayons.

Dans un réacteur de 2 titres, agité mécaniquement 500 g environ d'eau distillée, puis :
- 0,2 g de pyrophosphate acide de sodium,
- 0,5 g d'allantoïne,
- 100 g de glycérol à 98 %
- 35 g de Dapral T 210 ¹
- 5,6 g de lauryléthersulfate de sodium,
- 10 g de décylglucoside
- 13,7 g de peroxyde d'hydrogène à 70 %,
- 3,1 g d'acide acétique,
- 2,7 g d'une solution à 10 % d'acide peracétique,
- qsp 1000 g d'eau distillée.

(1): Agent viscosant commercialisé par la société Akzo.

A l'équilibre, la teneur en acide peracétique atteint 0,0030 % et la teneur en peroxyde d'hydrogène est de 1,0 %.

La viscosité de la solution est de 160 mPa.s.

Ces deux constituants sont stables dans cette formulation pendant plus d'un an.

Le pouvoir bactéricide de cette composition a été testé selon le protocole NFT 72-150 dans les conditions suivantes :
- température d'essai :ambiante
- bactéries testées :
   1) Staphylococcus aureus CNCM 53154, et
   2) Escherichia coli CNCM 54127
- temps de contact : 5 minutes
- dilution :1/2 et 1/4
- diluant :eau dure
- méthode d'essai : dilution - neutralisation
   Cette composition a permis de réduire d'au moins 10⁵ fois la population initiale des bactéries testées aussi bien quand ces bactéries sont Staphylococcus aureus qu'Escherichia Coli.

Le pouvoir fongicide de cette composition a été testé dans les conditions suivantes :
- levure testée : Candida albicans CNCM 11.80
- température d'essai : ambiante
- temps de contact : 15 minutes
- diluant : eau dure
- dilution : pur et dilué au 1/2
- méthode :filtration sur membrane 0,45 µm
   Cette composition a permis de réduire d'au moins 10⁴ fois la population de levure testée.

## Revendications

1. Composition aqueuse stable dans le temps, caractérisée en ce qu'elle comprend :
- un peroxyacide organique en une concentration inférieure à 0,09 % en poids ;
- et au moins un agent épaississant choisi parmi les composés de formule (VI) 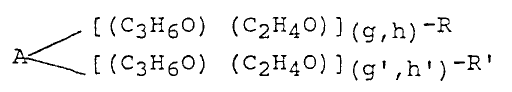 dans laquelle : [(C₃H₆O) (C₂H₄O)]_{(gi,hi)}, avec (gi, hi) désignant (g,h,) ou (g',h'), signifie qu'il s'agit d'un polymère statistique d'oxyde de propylène et d'oxyde d'éthylène contenant h mole d'oxyde d'éthylène et g mole d'oxyde de propylène, réparties de manière aléatoire dans la chaîne polymérique,
A désigne un reste de diisocyanate aliphatique, cycloaliphatique ou aromatique,
R et R', identiques ou différents, désignent un radical alkyle ou alcényle en C₈-C₃₀, de préférence en C₁₀-C₂₀,
gi et hi, identiques ou différents, sont tels que gi+hi est un nombre compris entre 20 et 200, et de préférence entre 60 et 120 ;
le rapport molaire hi/gi est compris entre 30/70 et 90/10, de préférence entre 50/50 et 90/10,
en une concentration telle que la viscosité de la composition est supérieure à 100 mPa.s (Brookfield LVT 2 à 20°C)

2. Composition selon la revendication 1 caractérisée en ce que le peroxyacide organique est choisi dans le groupe constitué par :
1) un monoperoxyacide de formule (I) :
R₁ - CO₃H (I)
dans laquelle R₁ représente un radical aliphatique en C₁-C₂₄, linéaire ou ramifié, saturé ou insaturé, un radical aryle ou un radical cycloalkyle en C₃ - C_{10,}
2) un diperoxyacide de formule (II) :
H₃OC - R₂ - CO₃H (II)
dans laquelle R₂ représente un reste aliphatique C₁ - C₂₄, linéaire ou ramifié, saturé ou insaturé, un radical arylène ou un radical cycloalkylène en C₃ - C₁₀,
chacun de R₁ ou de R₂ étant substitué ou non par un ou plusieurs groupes fonctionnels ou radicaux.

3. Composition selon l'une des revendications 1 et 2 caractérisée en ce que le peroxyacide organique est un composé de formule (I) dans laquelle R₁ est un radical alkyle en C₁ - C₁₂.

4. Composition selon la revendication 2, caractérisée en ce que le peroxyacide organique est l'acide peracétique.

5. Composition selon la revendication 1, caractérisée en ce que le peroxyacide organique est un imidoperoxyacide de formule (III) : 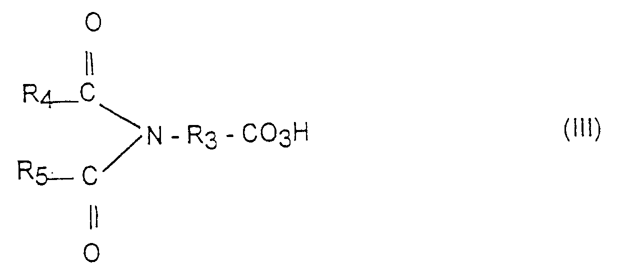 dans laquelle R₃ représente un reste aliphatique C₁-C₂₄, linéaire ou ramifié, saturé ou insaturé, un radical arylène ou un radical cycloalkylène en C₃-C₁₀, R₃ étant substitué ou non par un ou plusieurs groupes fonctionnels ou radicaux et R₄ et R₅ sont identiques ou différents et représentent un radical alkyle en C₁-C₈, linéaire ou ramifié, substitué ou non par un ou plusieurs groupes fonctionnels ou radicaux, ou R₄ et R₅ forment ensemble un reste aliphatique en C₂-C_{4,} linéaire, saturé ou mono-insaturé, substitué ou non par un radical alkyle en C₁-C₂₄ linéaire ou ramifié.

6. Composition selon la revendication 5 caractérisée en ce que le peroxyacide organique est un imidoperoxyacide comportant un hétérocycle azoté, de formule (IV) 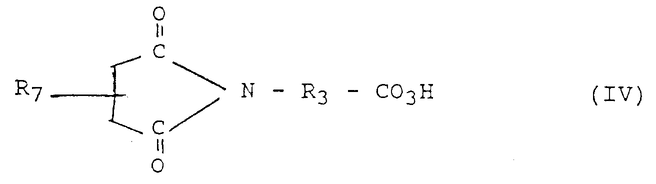 dans laquelle R₇ est un radical alkyle en C₁ - C₂₄, linéaire ou ramifié, substitué ou non par un ou plusieurs groupements fonctionnels ou radicaux ou R₇ est un groupe aryle, fusionné avec l'hétérocycle azoté, substitué ou non par un ou plusieurs groupes fonctionnels ou radicaux.

7. Composition selon la revendication 6 caractérisée en ce que dans la formule (IV), R₃ est un radical alkylène en C₁-C₂₀ et R₇ est un radical phénylène fusionné avec l'hétérocycle azoté.

8. Composition selon l'une des revendications 1 à 7, caractérisée en ce que la concentration en peroxyacide organique est comprise entre 0,001 % et 0,05 % en poids, et de préférence entre 0,002 % et 0,025% en poids.

9. Composition selon l'une quelconque des revendications 1 à 8, caractérisée en ce que A désigne un reste polyméthylène diisocyanate, ou toluylène diisocyanate, méthanediphénylène diisocyanate.

10. Composition selon la revendication 9, caractérisée en ce que A désigne le reste hexaméthylène diisocyanate et que R et R' désignent un radical lauryle ou un mélange de radicaux dérivés du suif.

11. Composition selon l'une des revendications 1 à 10 caractérisée en ce que le poids moléculaire du composé de formule (VI) est compris entre 1.000 et 20.000, de préférence entre 3.000 et 10.000.

12. Composition selon l'une des revendications 1 à 11, caractérisée en ce que la concentration en agent viscosant est telle que la viscosité de la composition est comprise entre 100 et 700 mPa.s (Brookfield LVT 2 à 20°C, de préférence entre 150 et 700mPa.s.

13. Composition selon l'une des revendications 1 à 12, caractérisée en ce qu'elle comporte en outre au moins un tensio-actif anionique, tels les alkyléthersulfates, les alkylbenzénesulfonates, les alkylsulfates, les oléfines sulfonates et les alkyléthercarboxylates, salifiés par un ou plusieurs cations.

14. Composition selon la revendication 13 caractérisée en ce que le tensio-actif anionique est le lauryléthersulfate de sodium.

15. Composition selon l'une des revendications 1 à 14, caractérisée en ce qu'elle comporte en outre au moins un tensio-actif non-ionique, tels les esters d'acides gras de sorbitan, les esters d'acides gras de sorbitan polyoxyéthylé et/ou polyoxypropylé et les alkylpolyglycosides de formule (VII)
R₉O (R₁₀O)_{Z} (G)x (VII)
où G est un reste d'un sucre réducteur contenant 5 ou 6 atomes de carbone, x est un un nombre compris entre 1 et 10, R₉ est un radical aliphatique, en C₆ - C₂₄, linéaire ou ramifié, saturé ou insaturé, R₁₀ est un groupe alkylène ayant de 2 à 4 atomes de carbone, et z est compris entre 0 et 20.

16. Composition selon la revendication 15 caractérisée en ce que dans la formule (VII) :
G est un reste de glucose ou de fructose,
x est compris entre 1 et 5,
R₉ est un radical alkyle comprenant de 8 à 18 atomes de carbone et z est égal à 0.

17. Utilisation d'une composition selon l'une des revendications 1 à 16 comme agent désinfectant et/ou nettoyant.

18. Utilisation d'une composition selon l'une des revendications 1 à 16, comme agent d'hygiène pour la désinfection de la peau ou des muqueuses de l'homme ou de l'animal.

19. Utilisation d'une composition selon l'une des revendications 1 à 16 comme agent d'hygiène pour la désinfection des trayons.

20. Utilisation d'une composition selon l'une des revendications 1 à 16 comme savon nettoyant et antiseptique.

## Claims

1. Aqueous composition which is stable with time, characterized in that it comprises :
- an organic peroxyacid at a concentration of less than 0.09 % by weight ;
- and at least one thickening agent selected from compounds of the formula (VI) : 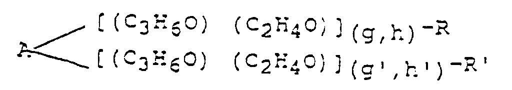 in which : [(C₃H₆O) (C₂H₄O)]_{(gi,hi)}, with (gi,hi) designating (g,h) or (g',h'), signifies that it consists of a statistical polymer of propylene oxide and ethylene oxide containing h mole of ethylene oxide and g mole of propylene oxide, distributed in a random manner in the polymer chain,
A designates an aliphatic, a cycloaliphatic or an aromatic diisocyanate residue,
R and R', identical or different, designate an alkyl or alkenyl radical with C₈-C₃₀, preferably with C₁₀-C₂₀,
gi and hi, identical or different, are such that gi+hi is a number which lies between 20 and 200, and preferably between 60 and 120,
the molar ratio hi/gi lies between 30/70 and 90/10, preferably between 50/50 and 90/10,
in a concentration such that the viscosity of the composition is above 100 mPa.s (Brookfield LVT 2 at 20°C).

2. Composition according to claim 1, characterized in that the organic peroxyacid is selected from the group consisting of :
1) a monoperoxyacid of formula (I) :
R₁ - CO₃H (I)
in which R₁ represents a saturated or unsaturated, linear or branched aliphatic radical with C₁ - C₂₄, an aryl radical or a cycloalkyl radical with C₃ - C₁₀,
2) a diperoxyacid of formula (II) :
H₃OC - R₂ - CO₃H (II)
in which R₂ represents a saturated or unsaturated, linear or branched aliphatic residue with C₁ - C₂₄, an arylene radical or a cycloalkylene radical with C₃ - C₁₀,
R₁ or R₂ each being substituted or not with one or more functional groups or radicals.

3. Composition according to either of claims 1 or 2, characterized in that the organic peroxyacid is a compound of formula (I) in which R₁ is an alkyl radical with C₁ - C₁₂.

4. Composition according to claim 2, characterized in that the organic peroxyacid is peracetic acid.

5. Composition according to claim 1, characterized in that the organic peroxyacid is an imidoperoxyacid of formula (III) : 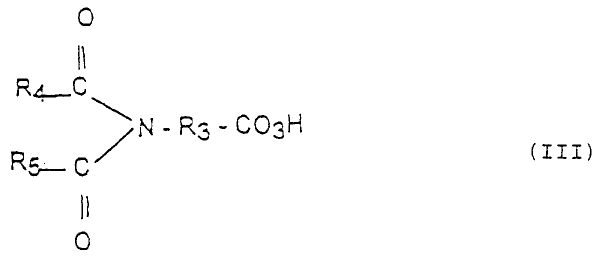 in which R₃ represents a saturated or unsaturated, linear or branched aliphatic residue with C₁ - C₂₄, an arylene radical or a cycloalkylene radical with C₃ - C₁₀, R₃ being substituted or not with several functional groups or radicals and R₄ and R₅ are identical or different and represent a linear or branched alkyl radical with C₁ - C₈, substituted or not with one or more functional groups or radicals, or R₄ and R₅ together form a saturated or unsaturated linear aliphatic residue with C₂ - C₄, substituted or not with a linear or branched alkyl radical with C₁-C₂₄.

6. Composition according to claim 5, characterized in that the organic peroxyacid is an imidoperoxyacid having a nitrogen-containing heterocyclic ring, of formula (IV) : 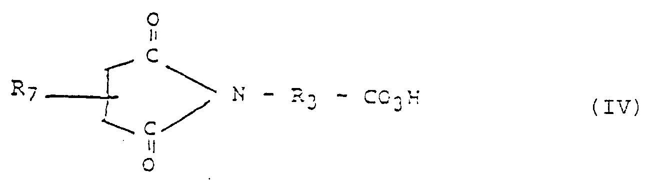 in which R₇ is a linear or branched alkyl radical with C₁ - C₂₄, substituted or not with one or more functional groups or radicals or R₇ is an aryl group fused with the nitrogen-containing heterocyclic ring, substituted or not with one or more functional groups or radicals.

7. Composition according to claim 6, characterized in that in the formula (IV), R₃ is an alkylene radical with C₁-C₂₀ and R₇ is a phenylene radical fused with the nitrogen-containing heterocyclic ring.

8. Composition according to one of claims 1 to 7, characterized in that the concentration of organic peroxyacid lies between 0.001 % and 0.05 % by weight, and preferably between 0.002 % and 0.025 % by weight.

9. Composition according to any one of claims 1 to 8, characterized in that A designates a polymethylene diisocyanate, toluylene diisocyanate or methanediphenylene diisocyanate residue.

10. Composition according to claim 9, characterized in that A designates the hexamethylene diisocyanate residue and in that R and R' designate a lauryl radical or a mixture of radicals derived from tallow.

11. Composition according to one of claims 1 to 10, characterized in that the molecular weight of the compound of formula (VI) lies between 1,000 and 20,000, preferably between 3,000 and 10,000.

12. Composition according to one of claims 1 to 11, characterized in that the concentration of thickening agent is such that the viscosity of the composition lies between 100 and 700 mPa.s (Brookfield LVT 2 at 20°C), preferably between 150 and 700 mPa.s

13. Composition according to one of claims 1 to 12, characterized in that it additionally includes at least one anionic surfactant, such as alkylethersulfates, alkylbenzenesulfonates, alkyl sulfates, olefin sulfonates and alkylethercarboxylates, converted to the salts with one or more cations.

14. Composition according to claim 13, characterized in that the anionic surfactant is sodium laurylethersulfate.

15. Composition according to one of claims 1 to 14, characterized in that it additionally includes at least one non-ionic surfactant, such as the fatty acid esters of sorbitan, the fatty acid esters of polyoxyethylated sorbitan and/or of polyoxypropylated sorbitan and alkylpolyglycosides of formula (VII)
R₉O (R₁₀O)_{z} (G)ₓ (VII)
where G is a residue of a reducing sugar containing 5 or 6 carbon atoms, x is a number which lies between 1 and 10, R₉ is a saturated or unsaturated, linear or branched aliphatic radical with C₆ - C₂₄, R₁₀ is an alkylene group having 2 to 4 carbon atoms and z lies between 0 and 20.

16. Composition according to claim 15, characterized in that in the formula (VII) :
G is a glucose or fructose residue,
x lies between 1 and 5,
R₉ is an alkyl radical having 8 to 18 carbon atoms and z is equal to 0.

17. Use of a composition according to one of claims 1 to 16 as a disinfecting and/or cleaning agent.

18. Use of a composition according to one of claims 1 to 16, as a hygienic agent for disinfecting the skin or mucous membranes of man or animals.

19. Use of a composition according to one of claims 1 to 16 as a hygienic agent for the disinfection of teats.

20. Use of a composition according to one of claims 1 to 16 as a cleaning and antiseptic soap.

## Patentansprüche

1. Dauerhaft stabile wäßrige Zusammensetzung, dadurch gekennzeichnet, daß sie umfaßt:
- eine organische Peroxysäure mit einer Konzentration von weniger als 0,09 Gew.-%;
- und mindestens ein Eindickungsmittel, ausgewählt aus den Verbindungen mit der Formel (VI) 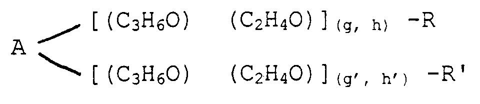 in der:
- [(C₃H₆O) (C₂H₄O)]_{(gi, hi)} - (gi, hi) bezeichnet (g, h) oder (g', h') - bedeutet, daß es sich um ein statistisches Polymer aus Propylenoxid und Ethylenoxid handelt, das h Mol Ethylenoxid und g Mol Propylenoxid enthält, die in der Polymerkette statistisch verteilt sind,
- A einen aliphatischen, cycloaliphatischen oder aromatischen Diisocyanatrest bezeichnet,
- R und R', die identisch oder verschieden sind, ein Alkyl- oder Alkinylradikal mit C₈-C₃₀, vorzugsweise mit C₁₀-C₂₀, bezeichnen,
- gi und hi, die identisch oder verschieden sind, so gewählt sind, daß gi+hi eine Zahl zwischen 20 und 200, vorzugsweise zwischen 60 und 120 ist,
das molare Verhältnis hi/gi zwischen 30/70 und 90/10, vorzugsweise zwischen 50/50 und 90/10 liegt,
und zwar mit einer Konzentration, so daß die Viskosität der Zusammensetzung größer als 100 mPa·s ist (Brookfield LVT 2 bei 20°C).

2. Zusammensetzung nach Anspruch 1, dadurch gekennzeichnet, daß die organische Peroxysäure aus der Gruppe ist, die besteht aus:
1) einer Monoperoxysäure mit der Formel(I):
R₁-CO₃H (I),
in der R₁ ein lineares oder verzweigtes, gesättigtes oder ungesättigtes, aliphatisches Radikal mit C₁ - C₂₄, ein Arylradikal oder ein Cycloalkylradikal mit C₃ - C₁₀ darstellt,
2) einer Diperoxysäure mit der Formel (II)
H₃OC-R₂-CO₃H (II),
in der R2 einen linearen oder verzweigte, gesättigten oder ungesättigten, aliphatischen Rest C₁ - C₂₄, ein Arylenradikal oder ein Cycloalkylenradikal mit C₃ - C₁₀ darstellt,
wobei R₁ oder R₂ jeweils durch eine oder mehrere Funktions- oder Radikalgruppen substituiert sein können.

3. Zusammensetzung nach Anspruch 1 oder 2, dadurch gekennzeichnet, daß die organische Peroxysäure eine Verbindung mit der Formel (I) ist, in der R₁ ein Alkylradikal mit C₁ - C₁₂ ist.

4. Zusammensetzung nach Anspruch 2, dadurch gekennzeichnet, daß die organische Peroxysäure Peressigsäure ist.

5. Zusammensetzung nach Anspruch 1, dadurch gekennzeichnet, daß die organische Peroxysäure eine Imidoperoxysäure mit der Formel (III) ist: 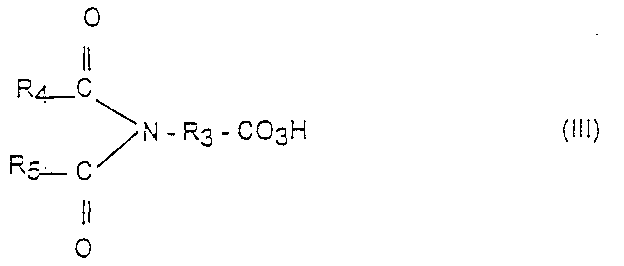 in der R₃ einen linearen oder verzweigten, gesättigten oder ungesättigten, aliphatischen Rest C₁ - C₂₄, ein Arylenradikal oder ein Cycloalkylenradikal mit C₃-C₁₀ darstellt, wobei R₃ durch eine oder mehrere funktionelle Gruppen oder Radikalgruppen ersetzt sein kann und R₄ und R₅ identisch oder verschieden sind und ein lineares oder verzweigtes Alkylradikal mit C₁ - C₈ darstellen, das durch eine oder mehrere funktionelle Gruppen oder Radikalgruppen substituiert sein kann, oder R₄ und R₅ gemeinsam einen linearen, gesättigten oder einfach ungesättigten aliphatischen Rest mit C₂-C₄ bilden, der durch ein lineares oder verzweigtes Alkylradikal mit C₁ - C₂₄ substituiert sein kann.

6. Zusammensetzung nach Anspruch 5, dadurch gekennzeichnet, daß die organische Peroxysäure eine Imidoperoxysäure ist, die eine heterocyclische Stickstoffverbindung mit der Formel (IV) umfaßt: 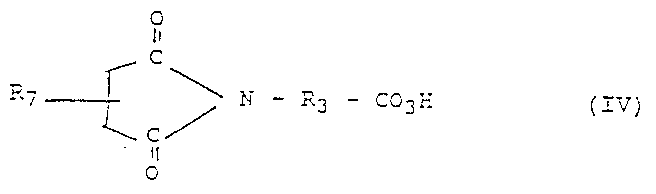 in der R₇ ein Alkylradikal mit C₁ - C₂₄ ist, das linear oder verzweigt ist und durch eine oder mehrere funktionelle Gruppen oder Radikalgruppen substituiert sein kann, oder R₇ eine mit der heterocyclischen Stickstoffverbindung verbundene Arylgruppe ist, die durch eine oder mehrere funktionelle Gruppen oder Radikalgruppen substituiert sein kann.

7. Zusammensetzung nach Anspruch 6, dadurch gekennzeichnet, daß in der Formel (IV) R₃ ein Alkylenradikal mit C₁ -C₂₀ ist und R₇ ein Phenylenradikal ist, das mit der heterocyclischen Stickstoffverbindung verbunden ist.

8. Zusammensetzung nach einem der Ansprüche 1 bis 7, dadurch gekennzeichnet, daß die Konzentration an organischer Peroxysäure zwischen 0,001 und 0,05 Gew.-%, vorzugsweise zwischen 0,002 und 0,025 Gew.-% liegt.

9. Zusammensetzung nach einem der Ansprüche 1 bis 8, dadurch gekennzeichnet, daß A einen Polymethylendiisocyanat-, Toluylendiisocyanat- oder Methandiphenylendiisocyanatrest bezeichnet.

10. Zusammensetzung nach Anspruch 9, dadurch gekennzeichnet, daß A den Hexamethylendiisocyanatrest bezeichnet und daß R und R' ein Laurylradikal oder ein Gemisch aus von Talg abgeleiteten Radikalen bezeichnen.

11. Zusammensetzung nach einem der Ansprüche 1 bis 10, dadurch gekennzeichnet, daß das Molekulargewicht der Verbindung mit der Formel (VI) zwischen 1.000 und 20.000, vorzugsweise zwischen 3.000 und 10.000 liegt.

12. Zusammensetzung nach einem der Ansprüche 1 bis 11, dadurch gekennzeichnet, daß die Konzentration an viskositätsbestimmendem Mittel so gewählt ist, daß die Viskosität der Zusammensetzung zwischen 100 und 700 mPa·s, vorzugsweise zwischen 150 und 700 mPa·s liegt (Brookfield LVT 2 bei 20°C).

13. Zusammensetzung nach einem der Ansprüche 1 bis 12, dadurch gekennzeichnet, daß sie zudem mindestens einen grenzflächenaktiven Stoff wie die Alkylethersulfate, die Alkylbenzolsulfonate, die Alkylsulfate, die Olefinsulfate und die Alkyletherperoxylate, die durch ein oder mehrere Kationen "versalzt" wurden, umfaßt.

14. Zusammensetzung nach Anspruch 13, dadurch gekennzeichnet, daß der anionische grenzflächenaktive Stoff Natriumlaurylethersulfat ist.

15. Zusammensetzung nach einem der Ansprüche 1 bis 14, dadurch gekennzeichnet, daß sie zudem mindestens einen nichtionischen grenzflächenaktiven Stoff wie Sorbitanfettsäureester, polyoxyethylen- und/oder polyoxypropylenhaltige Sorbitanfettsäureester und Alkylpolyglykoside mit der Formel (VII) enthält:
R₉O (R₁₀O)_{z}(G)ₓ (VII)
wobei G ein Rest eines 5 oder 6 Kohlenstoffatome enthaltenden, reduzierenden Zuckers, x eine Zahl zwischen 1 und 10 und R₉ ein lineares oder verzweigtes, gesättigtes oder ungesättigtes aliphatisches Radikal mit C₆ - C₂₄, R₁₀ eine Alkylengruppe mit 2 bis 4 Kohlenstoffatomen ist und z zwischen 0 und 20 liegt.

16. Zusammensetzung nach Anspruch 15, dadurch gekennzeichnet, daß in der Formel (VII)
G ein Glukose- oder Fruktoserest ist,
x zwischen 1 und 5 liegt,
R₉ ein Alkylradikal ist, das 8 bis 18 Kohlenstoffatome enthält, und z gleich 0 ist.

17. Verwendung einer Zusammensetzung nach einem der Ansprüche 1 bis 16 als Desinfektions- und/oder Reinigungsmittel.

18. Verwendung einer Zusammensetzung nach einem der Ansprüche 1 bis 16 als medizinisches Mittel zur Desinfektion der Haut oder der Schleimhäute des Menschen oder des Tiers.

19. Verwendung einer Zusammensetzung nach einem der Ansprüche 1 bis 16 als medizinisches Mittel zur Desinfektion von Zitzen.

20. Verwendung einer Zusammensetzung nach einem der Ansprüche 1 bis 16 als antiseptische Reinigungsseife.
